# EUROPEAN PATENT APPLICATION

(11) **EP 2 401 966 A1**
(43) Date of publication of application: **04.01.2012**
(21) Application number: 11172022.3
(22) Date of filing: 29.06.2011
(51) Int. Cl.: A61B 8/00, B06B 1/06, G10K 11/02

(54) **Ultrasound probe and ultrasound imaging apparatus**

(30) Priority: 30.06.2010 JP 2010148583
(71) Applicant: Kabushiki Kaisha Toshiba, Minato-ku Tokyo (JP); Toshiba Medical Systems Corporation, Otawara-shi, Tochigi-ken (JP)
(72) Inventor: Aoki, Minoru, Otawara-shi, Tochigi (JP); Shibamoto, Koichi, Otawara-shi, Tochigi (JP); Tsuzuki, Kentaro, Otawara-shi, Tochigi (JP); Takeuchi, Takashi, Otawara-shi, Tochigi (JP); Shikata, Hiroyuki, Otawara-shi, Tochigi (JP)
(74) Representative: Granleese, Rhian Jane

(57) **Abstract**

The ultrasound probe according to the present embodiment comprises ultrasound oscillators configured to transmit and receive ultrasound waves, an intermediate layer configured to have greater acoustic impedance than the ultrasound oscillators, a backing material configured to support the ultrasound oscillators, and a buffer layer. The ultrasound oscillators, the intermediate layer and the backing material are disposed in that order of the ultrasound oscillator, the intermediate layer and the backing material. The buffer layer, which has smaller acoustic impedance than the intermediate layer and the backing material, is disposed between the intermediate layer and the backing material.

## Description

### FIELD

The embodiments according to the present invention relate to an ultrasound probe and an ultrasound imaging apparatus.

### BACKGROUND

An ultrasound imaging apparatus sends ultrasound waves into a subject by means of an ultrasound probe, and the reflected waves arising from the gaps of acoustic impedance within the subject are received by the ultrasound probe. In addition, the ultrasound imaging apparatus, based on the reflected waves received by the ultrasound probe, produces an ultrasound image that represents the interior of the subject.

The ultrasound probe, for example, has a backing material, a plurality of piezoelectric transducers, an acoustic matching layer, and an acoustic lens. The backing material functions as a structural retainer, and furthermore, attenuates and absorbs excess ultrasound vibration components. The plurality of piezoelectric transducers is disposed in the scanning direction on the backing material. The piezoelectric transducers generate ultrasound waves by vibrating based on transmission signals, and produce reception signals upon receiving the reflected waves. One surface of the piezoelectric transducers is termed the "emitting surface", and the surface opposite to the emitting surface is termed the "rear surface". The backing material is provided on the rear surface of the piezoelectric transducers. The acoustic matching layer is provided on the emitting surface of the piezoelectric transducers. The acoustic matching layer moderates the mismatch in acoustic impedance between the piezoelectric transducers and the living body. The acoustic lens is provided on the acoustic matching layer. The acoustic lens converges the ultrasound waves. The ultrasound waves that are generated by the piezoelectric transducers are emitted through the acoustic matching layer and the acoustic lens.

A conventional ultrasound probe that has an intermediate layer between piezoelectric transducers and a backing material is known.

The acoustic impedance of the intermediate layer is higher than the acoustic impedance of the piezoelectric transducers. The thickness of the intermediate layer is about 1/4 of the wavelength of the ultrasound used. As the intermediate layer, gold, lead, tungsten, mercury or sapphire is used. The ultrasound waves emitted by the piezoelectric transducers to the rear surface side are reflected by the intermediate layer to the emitting surface side and emitted through the acoustic matching layer and the acoustic lens.

### BRIEF DESCRIPTION OF THE DRAWINGS

Fig. 1 is an oblique drawing of an ultrasound probe according to a first embodiment.
Fig. 2 is a sectional view of the ultrasound probe according to the first embodiment.
Fig. 3 is a sectional view of an ultrasound probe according to a second embodiment.
Fig. 4 is a schematic figure showing a simulation model.
Fig. 5A is a graph showing the result of the simulation.
Fig. 5B is a graph showing the result of the simulation.
Fig. 6 is a block diagram showing an ultrasound imaging apparatus according to the present embodiments.

### DETAILED DESCRIPTION

### [First embodiment]

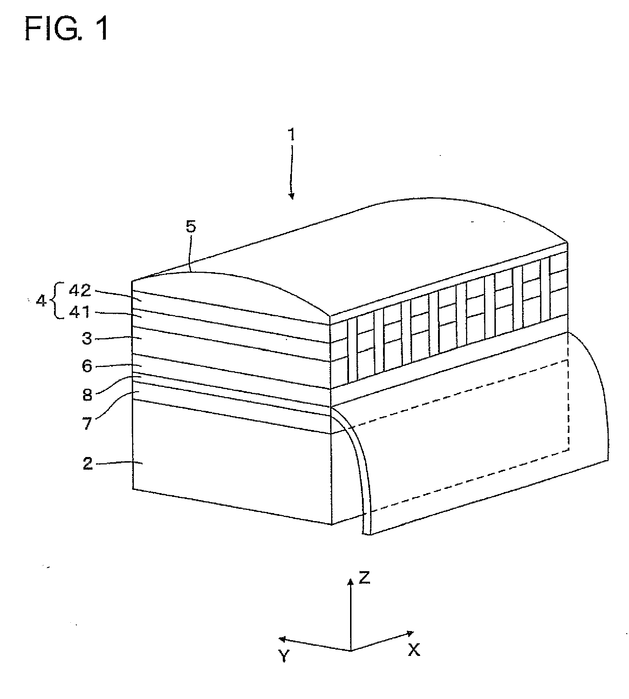

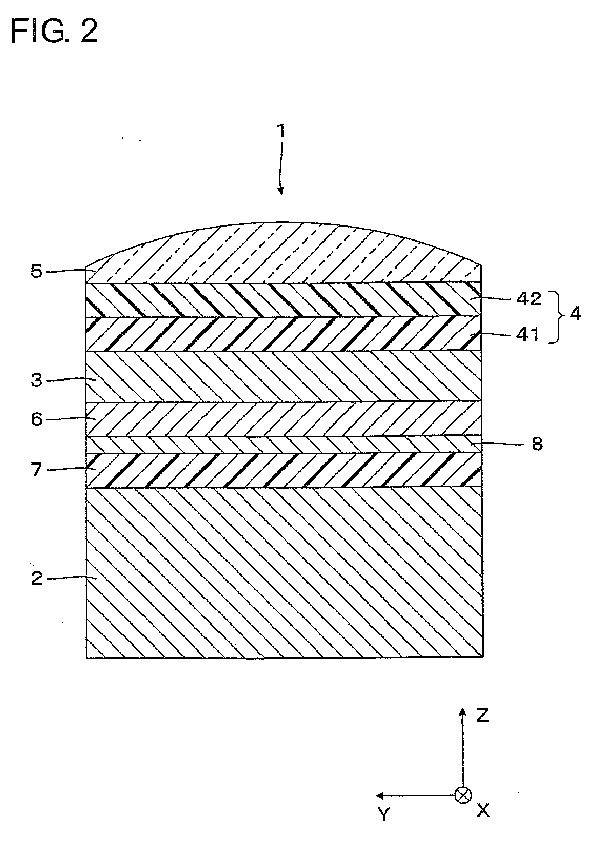

Referring to Fig. 1 and Fig. 2, the ultrasound probe according to the first embodiment is explained. The ultrasound probe 1 is provided with a head and a cable. In Fig. 1, the head of the ultrasound probe 1 is shown. Fig. 2 is a cross-section diagram in which the ultrasound probe 1 is cut along the Y-Z plane. Moreover, within the diagrams, the X direction is the scanning direction, and the Y direction is the slice direction, while the Z direction is the direction for transmission and reception of ultrasound waves. The X direction, the Y direction and the Z direction are respectively orthogonal to each other.

The ultrasound probe 1 according to the first embodiment is provided with a backing material 2, a plurality of ultrasound oscillators 3, a plurality of acoustic matching layers 4, an acoustic lens 5, an intermediate layer 6, a buffer layer 7, and a flexible printed circuit 8.

Each part is disposed in order of the backing material 2, buffer layer 7, flexible printed circuit 8, intermediate layer 6, ultrasound oscillators 3, acoustic matching layer 4, and the acoustic lens 5. ln other words, above the backing material 2, the buffer layer 7 is provided. Above the buffer layer 7, the flexible printed circuit (FPC) 8 is provided. Above the flexible printed circuit 8, the intermediate layer 6 is provided. Above the intermediate layer 6, a plurality of ultrasound oscillators 3 arranged in the scanning direction (the X direction in the figures) is provided. Above the ultrasound oscillators 3, a plurality of acoustic matching layers 4 arranged in the scanning direction is provided. Above the acoustic matching layers 4, the acoustic lens 5 is provided. The acoustic matching layers 4 comprise a first acoustic matching layer 41 provided above the ultrasound oscillators 3, and a second acoustic matching layer 42 provided above the first acoustic matching layer 41. The acoustic matching layers 4 may comprise a single layer, or may comprise a plurality of layers. A description of the cable is omitted. The acoustic impedance of the intermediate layer 6 is greater than the acoustic impedance of the ultrasound oscillators 3. The acoustic impedance of the buffer layer 7 is smaller than the acoustic impedance of the backing material 2 and the intermediate layer 6.

Below, each part of the ultrasound probe 1 is explained.

The backing material 2 supports the ultrasound oscillators 3.

The acoustic impedance of the backing material 2 is preferably, for example, 5 [Mrayl] or greater. The backing material 2 is preferably constructed from a metal such as aluminum, copper, titanium, zinc, tin, gold, or silver, or an alloy, metal carbide or metal oxide with those metals as a primary component. By using these materials in the backing material 2, it is possible to increase the rigidity of the backing material 2, and furthermore to increase the thermal conductivity of the backing material 2. By using a backing material 2 with a high thermal conductivity, it is possible to efficiently diffuse the heat generated by the ultrasound oscillators 3, through the backing material 2. Because it is possible to increase the thermal emission of the ultrasound probe 1 in this way, it becomes possible to transmit ultrasound waves with higher energy, and as a result, the sensitivity of the ultrasound probe 1 improves. The backing material 2 may be constructed from a rubber material that uses polybutadiene or chloroprene as a raw material.

The ultrasound oscillators 3, for example, are constructed from a ceramic such as lead zirconate titanate Pb(Zr,Ti)03, lithium niobate (LiNbO3), barium titanate (BaTi03), or lead titanate (PbTiO3). The upper surface and undersurface of the ultrasound oscillator 3 are respectively provided with electrodes, although this is not shown in the diagrams. The acoustic impedance of the ultrasound oscillators 3 is, for example, on the order of 30 [Mrayl]. The surface of the ultrasound oscillator 3 opposing the acoustic matching layer 4 is termed the emitting surface, and the surface on the opposite side of the emitting surface (the surface opposing the intermediate layer 6 is termed the "rear surface".

By adopting a structure with a plurality of layers for the acoustic matching layers 4, in conjunction with the acoustic lens 5, it is possible to inhibit the occurrence of signal loss from the difference in acoustic impedance with the body surface of the subject.

The acoustic lens 5 is in contact with the body surface of the subject, and mediates the transmission and reception of ultrasound waves. By means of this acoustic lens 5, an acoustic focal point in the slice direction (the Y direction in the figures) is formed at a prescribed depth from the body surface. Moreover, by switching the timing of the transmission and reception of the ultrasound waves by the plurality of ultrasound oscillators 3 disposed in the scanning direction, an acoustic focal point is formed in the scanning direction (X direction).

A plurality of ultrasound oscillators 3 is disposed in one line in the scanning direction (X direction), so the ultrasound probe 1 according to the present embodiment is a one-dimensional array probe.

Moreover, the ultrasound probe according to the present embodiment may be a two-dimensional array probe in which a plurality of ultrasound oscillators 3 is disposed in a two*-dimensional manner.

The intermediate layer 6 has greater acoustic impedance than the ultrasound oscillators 3. The acoustic impedance of the intermediate layer 6 is, for example, 50 to 100 [Mrayl]. The intermediate layer 6 is constructed, for example, from a conductive material. For example, the intermediate layer 6 may be constructed from a metal such as gold, lead, tungsten or mercury, or from sapphire. The intermediate layer 6 may also be constructed from a nonconductive material such as resin.

If a nonconductive material is used for the intermediate layer 6, through plating, etc., by forming metal on the surface of the intermediate layer 6, the intermediate layer 6 may be made conductive.

The thickness of the intermediate layer 6 is preferably about 1/4 of the wavelength (λ) of the ultrasound waves used. In this way, it is possible to match the phases of the ultrasound waves that are reflected at the intermediate layer 6 and return to the emitting surface side of the ultrasound oscillators 3, and the ultrasound waves that are emitted to the emitting surface side from the ultrasound oscillators 3.

The flexible printed circuit 8 comprises a conductive part and a substrate that supports the conductive part. The flexible printed circuit 8 is connected to the surface of the intermediate layer 6 opposing the backing material 2. The intermediate layer 6 is conductive, so the flexible printed circuit 8 applies a voltage to the ultrasound oscillators 3 through the intermediate layer 6. The acoustic impedance of the flexible printed circuit 8 is, for example, 5 to 10 [Mrayl]. Between the ultrasound oscillators 3 and the acoustic matching layers 4, a flexible printed circuit for extracting the ground signal, not shown in the diagrams, is provided. Moreover, the acoustic matching layers 4 may be made conductive, and between the acoustic matching layers 4 and the acoustic lens 5, a flexible printed circuit for extracting the ground signal may be provided.

The buffer layer 7 is disposed between the backing material 2 and the intermediate layer 6. ln the first embodiment, the flexible printed circuit 8 is disposed between the backing material 2 and the intermediate layer 6, and the buffer layer 7 is disposed between the backing material 2 and the flexible printed circuit 8. The acoustic impedance of the buffer layer 7 is smaller than the acoustic impedance of the backing material 2 and the intermediate layer 6. The acoustic impedance of the buffer layer 7 is, for example, 1 to 5 [Mrayl]. The buffer layer 7 is constructed, for example, from a high-polymer material such as a polyurethane material, a polyethylene material, or a polyimide material, or a silicon resin, or an epoxy resin, etc. The buffer layer 7 may be a structure with a plurality of laminated layers that are constructed from a high-polymer material, a silicon resin or an epoxy resin, etc. The thickness of the buffer layer 7 may be 1/16 or more of the wavelength (λ) of the ultrasound waves used.

### (Manufacturing method for the ultrasound probe 1)

Next, a manufacturing method for the ultrasound probe 1 is explained. First, the intermediate layer 6, the plate-form ultrasound oscillator, and the acoustic matching layers are laminated. ln particular, the plate-form ultrasound oscillator is joined to one surface of the intermediate layer 6 and the acoustic matching layer is joined to the surface of the ultrasound oscillator on the opposite side of the surface facing the intermediate layer 6. The flexible printed circuit 8 is joined to the surface of the intermediate layer 6 on the opposite side of the surface facing the ultrasound oscillator. Next, the buffer layer 7 is joined to the surface of the flexible printed circuit 8 on the opposite side of the surface facing the intermediate layer 6. The backing material 2 is joined to the surface of the buffer layer 7 on the opposite side of the surface facing the flexible printed circuit 8. For each joint, for example, an epoxy adhesive may be used. Moreover, the buffer layer 7 may be joined in advance to the flexible printed circuit 8 and the backing material 2. In the manner above, in the order of the acoustic matching layer, plate-form ultrasound oscillator, intermediate layer 6, flexible printed circuit 8, buffer layer 7, and backing material 2, a structure is produced in which these parts are laminated. By making multiple cuts in the plate-form ultrasound oscillators and the acoustic matching layer along the scanning direction (X direction) at the surface including the slice direction (Y direction) and the transmission and reception direction (Z direction), a plurality of ultrasound oscillators 3 and a plurality of acoustic matching layers 4 are obtained. By disposing the acoustic lens 5 on the acoustic matching layer 4, the ultrasound probe 1 is produced.

ln accordance with the ultrasound probe 1 that has the abovementioned configuration, the buffer layer 7, which has an acoustic impedance smaller than the backing material 2 and the intermediate layer 6, is disposed between the backing material 2 and the intermediate layer 6, so it is possible to inhibit the propagation of ultrasound waves from the ultrasound oscillators 3 to the backing material 2. ln other words, ultrasound that is emitted from the ultrasound oscillators 3 to the rear surface side (to the intermediate layer 6 side) are reflected to the emitting surface side of the ultrasound oscillators 3 by the intermediate layer 6, which has a higher acoustic impedance than the ultrasound oscillators 3. On the other hand, some of the ultrasound waves that are emitted to the rear surface side propagate through the intermediate layer 6 and head toward the side of the backing material 2. ln the first embodiment, the buffer layer 7, which has an acoustic impedance smaller than the backing material 2 and the intermediate layer 6, is disposed between the backing material 2 and the intermediate layer 6, so the ultrasound waves that have passed through the intermediate layer 6 are reflected by the buffer layer 7. ln this manner, it is possible to inhibit the propagation of ultrasound waves from the ultrasound oscillators 3 to the backing material 2. As a result, because the reflection of ultrasound waves by the backing material 2 is inhibited, it is possible to inhibit the propagation of ultrasound waves from the backing material 2 to the emitting surface side of the ultrasound oscillators 3. Because it is possible to reduce the effect of the ultrasound waves reflected by the backing material 2 in this way, ultrasound images with few artifacts can be obtained without degradation of the acoustic characteristics of the ultrasound probe 1.

Moreover, because the buffer layer 7 is disposed between the backing material 2 and the intermediate layer 6, the backing material 2 does not necessarily need to have a function to attenuate ultrasound waves. !n other words, the backing material 2 may have the function of supporting the ultrasound oscillators 3. ln this way, the constraints pertaining to the materials used for the backing material 2 are reduced, so it is possible to use a material with high thermal conductivity for the backing material 2. Therefore, as described above, it is possible to use a metal, metal carbide or metal oxide as a material for the backing material 2, raising the thermal conductivity of the backing material 2.

ln this way, heat generated by the ultrasound oscillators 3 can be more efficiently diffused through the backing material 2, so it is possible to transmit ultrasound waves with higher energy.

Moreover, by using the buffer layer 7, it is possible to attenuate the mechanical vibration of the ultrasound oscillators 3 or the intermediate layer 6. The ultrasound oscillators 3 mechanically vibrate when ultrasound waves are generated. This vibration is propagated to the intermediate layer 6, the flexible printed circuit 8, and the backing material 2, and sometimes cross talk occurs. As described above, a material with low acoustic impedance is used for the buffer layer 7, so the Poisson's ratio of the buffer layer 7 is high. By disposing a buffer layer 7 with a relatively high Poisson's ratio, it is possible to buffer and attenuate the vibration of the ultrasound oscillators 3 or intermediate layer 6. As a result the occurrence of cross talk can be inhibited.

Moreover, as described above, the thickness of the buffer layer 7 can be set according to the wavelength (λ) of the ultrasound waves.

Here, by setting the thickness of the buffer layer 7 to the prescribed thickness or more, the propagation of ultrasound waves from the ultrasound oscillators 3 to the backing material 2 can be more effectively inhibited. In addition, the vibration of the ultrasound oscillators 3 or the intermediate layer 6 can be more effectively buffered and attenuated.

However, in the setting of the thickness of the buffer layer 7, the thermal emission characteristics of the ultrasound probe, and in particular the thermal conductivity of the buffer layer 7, is preferably taken into consideration.

Moreover, in the manufacturing method of the ultrasound probe provided as an example above, in the order of acoustic matching layer, plate-form ultrasound oscillator, intermediate layer 6, flexible printed circuit 8, buffer layer 7 and backing material 2, a structure is produced in which these parts are laminated. In this structure, the plate-form ultrasound oscillator and acoustic matching layer are multiply cut. In the case of such a manufacturing method, it is preferable to set the thickness of the buffer layer 7 so that the structure cutting process is not disturbed. In other words, this is because if the buffer layer 7, which has a relatively high Poisson's ratio, is made too thick, there is sometimes a negative impact on the structure cutting process.

Therefore, it is possible to set the thickness of the buffer layer 7, taking into account, as necessary, the perspectives of the inhibition of the propagation of ultrasound waves to the backing material 2, the attenuation of vibration, the thermal emission characteristics, the effect on the manufacturing processes, etc.

### [Second embodiment]

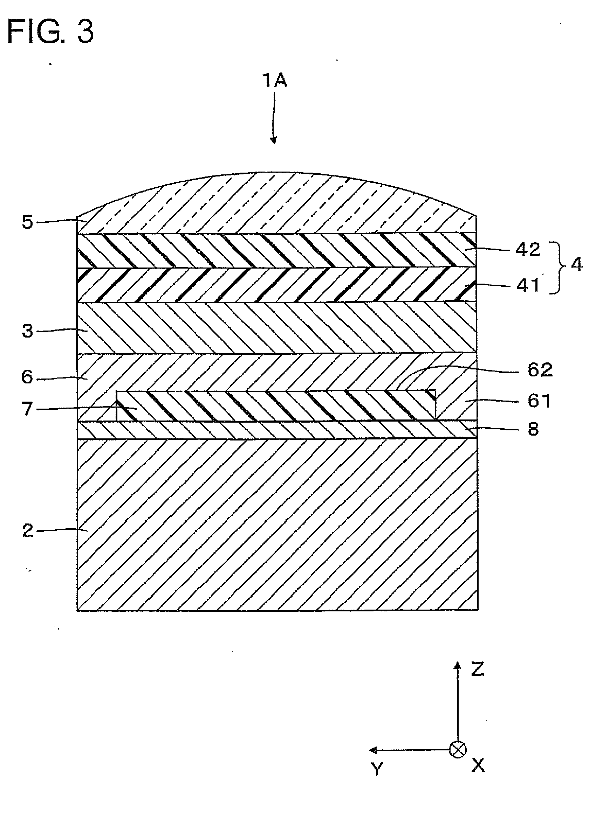

Referring to Fig. 3, the ultrasound probe according to a second embodiment is explained. Fig. 3 is a cross-section diagram cut along the Y-Z plane of the ultrasound probe 1A according to the second embodiment. In the first embodiment, the buffer layer 7 is disposed between the backing material 2 and the flexible printed circuit 8, but in the second embodiment, the position of the buffer layer 7 is changed.

In the second embodiment, the buffer layer 7 is disposed between the intermediate layer 6 and the flexible printed circuit 8.

Moreover in the ultrasound probe 1A according to the second embodiment, the position of the buffer layer 7 and the shape of the intermediate layer 6 are different from the ultrasound probe 1 according to the first embodiment, but other than those, the configuration is the same as the configuration of the ultrasound probe 1. Below, the intermediate layer 6 and the buffer layer 7 are explained.

The intermediate layer 6 is provided with a concave portion 62 on the surface opposing the backing material 2 (the surface on the opposite side of the surface facing the ultrasound oscillators 3). For example, the intermediate layer 6 has a concave portion 62 at a location corresponding to the effective aperture of the ultrasound waves in the surface opposing the backing material 2. The shape and size of the concave portion 62 are almost the same as the shape and size of the buffer layer 7. In other words, the buffer layer 7 according to the second embodiment has almost the same shape and size as the concave portion 62. The buffer layer 7 is disposed within the concave portion 62 of the intermediate layer 6. Moreover, the intermediate layer 6 has a protruding portion 61 surrounding the concave portion 62 in the periphery of the concave portion 62. By disposing the buffer layer 7 within the concave portion 62, it is surrounded by the protruding portion 61. Moreover, the flexible printed circuit 8 is disposed between the intermediate layer 6 and the buffer layer 7, and the backing material 2. The protruding portion 61 surrounds the buffer layer 7 and extends toward the flexible printed circuit 8, and the tip of the protruding portion 61 is joined to the flexible printed circuit 8. In this way, the intermediate layer 6 and the flexible printed circuit 8 are electrically connected.

According to the ultrasound probe 1A that has the abovementioned structure, while raising the effectiveness of the buffer layer 7, it is possible to apply a voltage to the ultrasound oscillators 3 from the flexible printed circuit 8 through the intermediate layer 6. In other words, as described above, in order to raise the effectiveness of the buffer layer 7, the buffer layer 7, without the flexible printed circuit 8 being sandwiched in between, is preferably disposed directly below the intermediate layer 6. As an example, it is preferable that each part is disposed in order of the intermediate layer 6, buffer layer 7, flexible printed circuit 8. However, depending on the material used for the buffer layer 7, the buffer layer 7 may not be conductive. If a buffer layer 7 that is not conductive is disposed between the intermediate layer 6 and the flexible printed circuit 8, it is difficult to obtain an electrical connection between the flexible printed circuit 8 and the ultrasound oscillators 3. Hence, as in the ultrasound probe 1A according to the second embodiment, a concave portion 62 is provided in the intermediate layer 6, the buffer layer 7 is disposed in the concave portion 62, and in addition, the protruding portion 61 of the intermediate layer 6 is joined to the flexible printed circuit 8. In this way, it is possible to dispose the buffer layer 7 directly below the intermediate layer 6, so it is possible to raise the effectiveness of the buffer layer 7. Moreover, the protruding portion 61 of the intermediate layer 6 and the flexible printed circuit 8 are joined, so the intermediate layer 6 and the flexible printed circuit 8 are electrically connected, and it is possible to apply a voltage to the ultrasound oscillators 3 from the flexible printed circuit 8 through the intermediate layer 6.

### (Simulation)

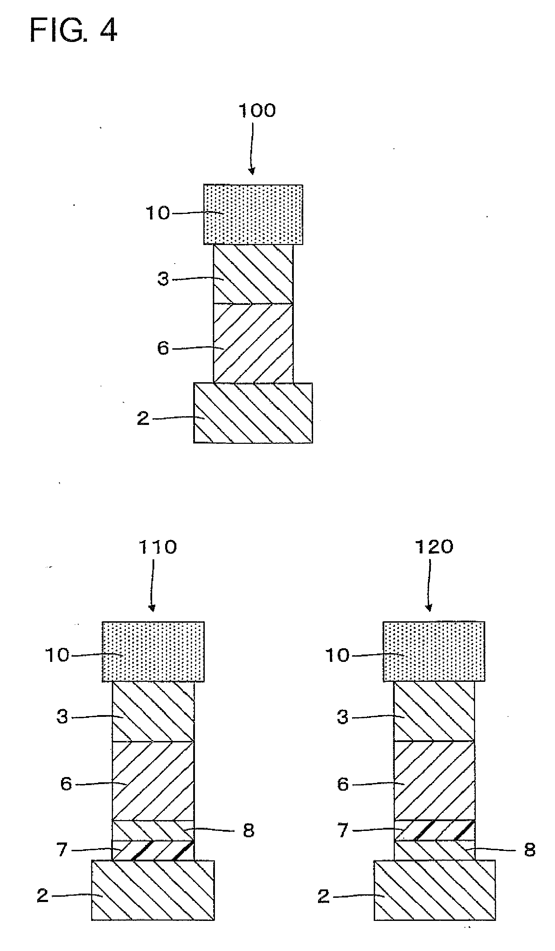

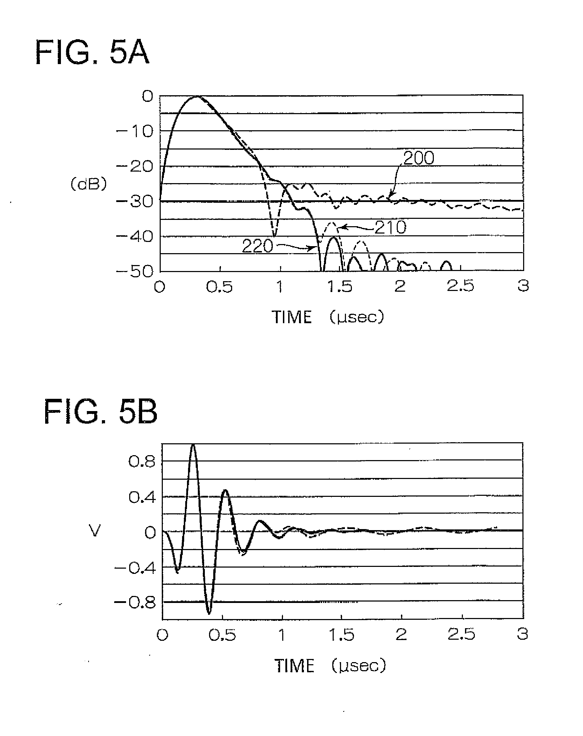

Taking the ultrasound probe 1 according to the first embodiment and the ultrasound probe 1A according to the second embodiment as subjects, an acoustic simulation was performed by means of finite element analysis. The simulation results are shown in Fig. 4 and Fig. 5A and Fig. 5B, Models having the ultrasound oscillators 3, the intermediate layer 6, the flexible printed circuit 8, the buffer layer 7, and the backing material 2 were assumed, and by conducting a simulation about those models, the changes by time of the sound pressure on the front of the ultrasound oscillators 3 were derived.

Each model is shown in Fig. 4. Model 100 is a model of an ultrasound probe without the buffer layer 7. Model 100 has the backing material 2, the intermediate layer 6, and the ultrasound oscillator 3, each part being disposed in that order. Model 110 is equivalent to the ultrasound probe 1 according to the first embodiment. Model 110 has the backing material 2, the buffer layer 7, the flexible printed circuit 8, the intermediate layer 6, and the ultrasound oscillators 3, each part being disposed in that order. Model 120 is equivalent to the ultrasound probe 1A according to the second embodiment. Model 120 has the backing material 2, the flexible printed circuit 8, the buffer layer 7, the intermediate layer 6, and the ultrasound oscillators 3, each part being disposed in that order. Moreover, in each model, water 10 is disposed in the front of the ultrasound oscillators 3.

The parameters used in the simulation are shown below. Za is the acoustic impedance, and λ is the wavelength of the ultrasound waves.
Central frequency of the ultrasound waves: 3[MHz]
Ultrasound oscillators 3: Za=about 30[Mrayl], thickness=about λ/4
Intermediate layer 6: Za=about 80[Mrayl], thickness=about λ/4
Backing material 2: Za=about 20[Mrayl], thickness=about 2[mm]
Buffer layer 7: Za=about 2[Mrayl], thickness=about λ/8
Flexible printed circuit 8: Za=about 5[Mrayl], thickness=about 0.1mm

The results of the simulation are shown in Fig. 5A and Fig. 5B.

Fig. 5A shows the sound pressure distribution [dB], while Fig. 5B shows the sound pressure values V. The horizontal axis of each graph represents time. Graph 200 is the result of the model 100 simulation. Graph 210 is the result of the model 110 simulation. Graph 220 is the result of the model 120 simulation. According to the sound pressure distribution, from about 1.5[µsec] onward, a difference arises in the strength of the waveforms. In the model 100, which has no buffer layer 7, as shown in graph 200, the result is that the convergence of the waveforms is not satisfactory. This result is the cause of a decrease in range resolution in an ultrasound imaging apparatus, and causes the degradation of image quality in ultrasound images. In contrast, according to model 110 and model 120, which have the buffer layer 7, as shown in graph 210 and graph 220, the waveform convergence has improved compared to model 100. This result, due to the buffer layer 7 with low acoustic impedance being disposed between the intermediate layer 6 and the backing material 2, is attributed to reducing the reflection of the ultrasound waves from the backing material 2.

Moreover, as shown in graph 210 and graph 220, whether the buffer layer 7 is disposed on the side of the backing material 2 against the flexible printed circuit 8 (the mode of model 110), or whether it is disposed on the side of the intermediate layer 6 (the mode of model 120), a similar effect is obtained.

### (Ultrasound imaging apparatus)

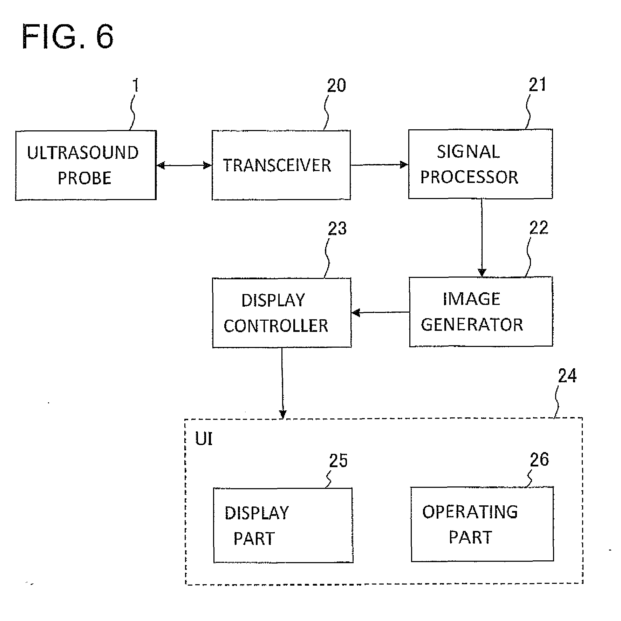

Referring to Fig. 6, the ultrasound imaging apparatus according to the present embodiment is explained. The ultrasound imaging apparatus according to the present embodiment is provided with the ultrasound probe 1 according to the first embodiment, a transceiver 20, a signal processor 21, an image generator 22, a display controller 23, and a User Interface (Ul) 24. Moreover, the ultrasound imaging apparatus according to the present embodiment, instead of the ultrasound probe 1, may be provided with the ultrasound probe 1A according to the second embodiment.

### (Transceiver 20)

The transceiver 20 is provided with a transmission part and a reception part, not shown in the diagrams. The transceiver 20 supplies electrical signals to the ultrasound probe 1, generates ultrasound waves, and receives echo signals received by the ultrasound probe 1.

The transmission part supplies electrical signals to the ultrasound probe 1, and transmits ultrasound waves that have been beam formed at a prescribed focal point (transmission beam forming).

The transmission part is provided with, for example, a clock generator, a transmission delay circuit, and a pulsar circuit, which are not shown in the diagrams. The clock generator generates a clock signal that determines the transmission timing and transmission frequency of ultrasound signals. The transmission delay circuit, in accordance with a delay time for focusing, which is for focusing ultrasound waves at a prescribed depth, and a delay time for deflection, which is for sending ultrasound waves in a prescribed direction, at the time of transmitting ultrasound waves, a delay is introduced and the transmission focus is performed. The pulsar circuit has pulsars with the same number as that of the individual channels corresponding to the ultrasound oscillators 3. The pulsar circuit, according to the transmission timing into which a delay has been introduced, generates driving pulses, and supplies them to each ultrasound oscillator 3 of the ultrasound probe 1.

The reception part receives the echo signals that the ultrasound probe 1 receives, and by performing a delay process on the echo signals, converts the analog received signals to phased (reception beam-formed) digital received signals. The reception part is provided with, for example, a preamplifier circuit, an A/D converter, a receiving delay circuit, and an adder, which are not shown in the figures. The preamplifier circuit amplifies the echo signals output from each ultrasound oscillator 3 of the ultrasound probe 1 to each receive channel. The A/D converter converts the amplified echo signals to digital signals. The receiving delay circuit applies a delay time, which is necessary for determining the reception directionality, to the echo signal that has been converted to digital signals. In particular, the receiving delay circuit applies to the digital echo signal a delay time for focusing, which is for focusing ultrasound waves at a prescribed depth, and a delay time for deflection, which is for setting the reception directionality for a prescribed direction. The adder computes the echo signal to which the delay time has been introduced. By this addition, the reflection component from the direction corresponding to the reception directionality is enhanced. In other words, by means of the receiving delay circuit and the adder, the received signal obtained from the prescribed direction is phased and added.

### (Signal processor 21)

The signal processor 21 has a B mode processor. The B mode processor performs visualization of the echo amplitude information, and produces B mode ultrasound raster data from the echo signals. In particular, the B mode processor performs a bandpass filter processing on the received signals, and subsequently, detects envelope curves of the output signals and performs compression processing on the detected data by means of logarithmic transformation. Moreover, the signal processor 21 may have a CFM (Color Flow Mapping) processor. The CFM processor performs visualization of the blood flow information, and produces color ultrasound raster data. The blood flow information includes such as velocity, distribution and power, and the blood flow information is obtained as binary information. Moreover, the signal processor 21 may have a Doppler processor. The Doppler processor, by performing phase detection on the received signals, extracts the Doppler shift frequency component, and by performing FFT processing, produces a Doppler frequency distribution that expresses the blood flow velocity.

### (image generator 22)

The image generator 22 produces an ultrasound image data based on the data output from the signal processor 21. The image generator 22 is provided with, for example, a DSC (Digital Scan Converter). The image generator 22 converts the data after signal processing, which is expressed by the signal rows of the scanning line, to the image data expressed by the Cartesian coordinate system (scan conversion processing). For example, the image generator 22, by performing scan conversion processing on the B mode ultrasound raster data, on which signal processing has been has been performed by the B mode processor, produces B mode image data expressing the shape of the subject's tissue.

### (Display controller 23)

The display controller 23, based on ultrasound imaging data produced by the image generator 22, displays the ultrasound image on the display part 25.

### (User Interface (Ul) 24)

The user Interface (Ul) 24 is provided with a display part 25 and an operating part 26. The display part 25 is a monitor such as a CRT or a liquid crystal display, and displays ultrasound images such as cross-sectional images, etc., on the screen. The operating part 26 is configured with a pointing device such as a joystick or trackball, switches, various buttons, a keyboard, or a TCS (Touch Command Screen).

The image generator 22 and the display controller 23 may respectively be constructed by a processing device such as a CPU, GPU, or ASlC, which are not shown in the figures, and a memory device such as ROM, RAM or HDD, which are not shown in the figures. In the memory device, an image generation program for executing functions of the image generator 22, and a display control program for executing functions of the display controller 23 are stored.

By having processing devices such as CPU execute each program stored in the memory device, the functions of each part are executed.

According to the ultrasound imaging apparatus having the abovementioned configuration, because it is provided with the ultrasound probe 1 according to the first embodiment or the ultrasound probe 1A according to the second embodiment, without degradation of the acoustic characteristics of the ultrasound probe 1 or the ultrasound probe 1A, it is possible to produce ultrasound images with few artifacts.

While certain embodiments have been described, these embodiments have been presented by way of example only, and are not intended to limit the scope of the inventions. Indeed, the novel systems described herein may be embodied in a variety of their forms; furthermore, various omissions, substitutions and changes in the form of the systems described herein may be made without departing from the spirit of the inventions. The accompanying claims and their equivalents are intended to cover such forms or modifications as would fall within the scope and spirit of the inventions.

## Claims

1. An ultrasound probe, comprising:
ultrasound oscillators configured to send and receive ultrasound waves;
an intermediate layer configured to have a greater acoustic impedance than the ultrasound oscillators; and
a backing material configured to support the ultrasound oscillators,
the ultrasound oscillators, the intermediate layer and the backing material being disposed in order of the ultrasound oscillators, the intermediate layer and the backing material,
and further comprising a buffer layer configured to have a smaller acoustic impedance than the intermediate layer and the backing material, and be disposed between the intermediate layer and the backing material.

2. The ultrasound probe according to claim 1,
wherein the acoustic impedance of the buffer layer is from 1 to 5 [Mrayl].

3. The ultrasound probe according to claim 1,
further comprising a substrate configured to be connected to a surface of the intermediate layer opposing the backing material, and be for applying a voltage to the ultrasound oscillators,
wherein the intermediate layer is conductive, and
the buffer layer is placed between the substrate and the backing material.

4. The ultrasound probe according to claim 2,
further comprising a substrate configured to be connected to a surface of the intermediate layer opposing the backing material, and be for applying a voltage to the ultrasound oscillators,
wherein the acoustic matching layer is conductive, and
the buffer layer is placed between the substrate and the backing material.

5. The ultrasound probe according to claim 1,
further comprising a substrate configured to be for applying a voltage to the ultrasound oscillator, and be disposed between the intermediate layer and the backing material,
wherein:
the intermediate layer is conductive, and further comprises a concave portion in the surface opposing the backing material;
the buffer layer is positioned within the concave portion of the intermediate layer; and
the periphery of the concave portion of the intermediate layer and the substrate are connected.

6. The ultrasound probe according to claim 2,
further comprising a substrate configured to be for applying a voltage to the ultrasound oscillator, and be disposed between the intermediate layer and the backing material,
wherein:
the intermediate layer is conductive, and further comprises a concave portion in the surface opposing the backing material;
the buffer layer is positioned within the concave portion of the intermediate layer; and
the periphery of the concave portion of the intermediate layer and the substrate are connected.

7. The ultrasound probe according to claim 1,
wherein the backing material is formed from a metal, a metal carbide or a metal oxide.

8. An ultrasound imaging apparatus, comprising:
the ultrasound probe according to any of claims 1 to 7; and
an image generator configured to generate an ultrasound image based on the signals received by the ultrasound probe.
